# FASCICULE DE BREVET EUROPEEN

(11) **EP 0 575 502 B1**
(45) Date de publication et mention de la délivrance du brevet: **20.11.1996**
(21) Numéro de dépôt: 92908338.4
(22) Date de dépôt: 13.03.1992
(51) Int. Cl.: A61K 9/16, A61K 9/22, A61K 9/70, A61K 9/20, A61K 47/34

(54) **IMPLANT BIORESORBABLE A BASE D'ACIDE POLYLACTIQUE CONTENANT UN ANTIBIOTIQUE**
BIORESORBIERBARES IMPLANTAT BESTEHEND AUS POLYMILCHSÄURE UND ENTHALTEND ANTIBIOTIKA
POLYLACTIC ACID-BASED IMPLANT SUSCEPTIBLE OF BIORESORPTION CONTAINING AN ANTIBIOTIC

(30) Priorité: 14.03.1991 FR 9103110
(43) Date de publication de la demande: 29.12.1993
(73) Titulaire: CENTRE NATIONAL DE LA RECHERCHE SCIENTIFIQUE (CNRS), 75794 Paris Cédex 16 (FR)
(72) Inventeur: VERT, Michel, F-76130 Mont-Saint-Aignan (FR); MAUDUIT, Jacques, F-76730 Bacqueville-en-Caux (FR); BUKH, Niels, DK-2900 Hellerup (DK)
(74) Mandataire: Tonnellier, Jean-Claude
(86) Numéro de dépôt international: FR9200231
(87) Numéro de publication internationale: WO9216193

(56) Documents cités:
- EP-A- 0 049 068
- EP-A- 0 251 680
- EP-A- 0 374 531
- WO-A-90/15586
- DE-A- 3 444 832
- GB-A- 2 091 554

## Description

La présente invention a pour objet une composition pharmaceutique implantable, biorésorbable à base de poly(acide lactique), destinée notamment à mettre en oeuvre une antibiothérapie interne locale.

On sait que l'industrie pharmaceutique recherche actuellement de nouvelles formes médicamenteuses implantables, libérant progressivement le principe actif, dans le but de remédier aux défauts des formes galéniques classiques, et en particulier d'éviter la nécessité d'administrations répétées.

Les systèmes à libération contrôlée sont généralement des microcapsules, des microsphères, ou des implants massifs de formes diverses, par exemple cylindriques (aiguilles). Dans ces systèmes, le principe actif est généralement dispersé dans une matrice polymère biorésorbable.

Parmi les polymères biorésorbables, les poly(acides lactiques) sont les plus utilisés. La littérature fournit des exemples variés d'application de tels systèmes, notamment à la chimiothérapie anti-cancéreuse et à l'hormonothérapie.

Dans le domaine de l'antibiothérapie, on a déjà décrit des microsphères de poly(acide lactique) contenant de l'érythromycine. Ces microsphères sont administrées par injection parentérale, et agissent par voie systémique, en libérant progressivement l'antibiotique, tout en réduisant l'irritation des tissus au point d'injection ; voir P.K. Gupta et al, 5th Congr.Int.Technol.Pharm., 4, 73-79, 1989.

Toutefois, les microsphères ne constituent pas une forme d'administration satisfaisante, d'une part car elles contiennent des résidus de tensioactifs utilisés dans leur préparation, et d'autre part car elles contiennent généralement en surface une pellicule de polymère exempte de substance active qui retarde dans un premier temps la libération de cette substance.

La présente invention a pour objet une composition pharmaceutique implantable destinée en particulier à mettre en oeuvre une antibiothérapie non pas générale, mais locale, essentiellement limitée aux sites opératoires, afin de prévenir et/ou de guérir les infections locales qu'il faut toujours redouter après une intervention chirurgicale.

Pour éviter ces infections, on utilise actuellement l'antibiothérapie systémique avec des antibiotiques à large spectre administrée par voie parentérale. On connaît les inconvénients de l'antibiothérapie générale : injection douloureuse avec irritation des tissus, toxicité rénale ou hépatique, et nécessité d'administrations répétées.

La présente invention a pour objet de remédier à ces inconvénients en mettant à la disposition du chirurgien une composition biorésorbable, destinée à être implantée sur le site opératoire, avant suture de la plaie chirurgicale, et permettant de mettre en oeuvre une antibiothérapie interne locale par libération progressive de la substance antibiotique. Bien entendu, la composition biorésorbable de l'invention, destinée à assurer une antibiothérapie locale concernant le site opératoire, est mise en place principalement au contact des tissus mous internes accessibles par la plaie chirurgicale. Elle peut aussi être placée à la surface de contact entre un implant osseux massif (notamment pièce d'ostéosynthèse) et la cavité osseuse destinée à recevoir cet implant.

Pour la commodité d'une telle utilisation, la composition de l'invention se présente sous la forme d'une poudre ou d'un film mince. La poudre est une poudre obtenue par broyage d'une dispersion de l'antibiotique dans la matrice polymère, ce qui permet d'éviter les inconvénients, signalés ci-dessus, des microsphères.

On a en effet découvert que les compositions sous forme de poudre broyée ou de film mince permettent d'obtenir un profil de libération satisfaisant, avec une phase initiale de libération rapide et importante (ou "burst") de l'antibiotique présent en surface, permettant d'obtenir une dose d'attaque appropriée, suivie d'une phase de libération plus lente de l'antibiotique sur une durée suffisamment longue, par exemple une dizaine de jours.

Dans les compositions de l'invention, la dégradation de la matrice polymère est relativement lente par rapport à la période de libération de l'antibiotique, afin que cette libération soit contrôlée par des phénomènes de dissolution-diffusion de l'antibiotique dans le milieu externe, et non par suite de la dégradation de la matrice.

Une autre caractéristique des compositions de l'invention est que l'antibiotique est ajouté sous la forme de particules ayant des dimensions contrôlées, ce qui permet de mieux maîtriser la reproductibilité et la durée du phénomène de libération de l'antibiotique.

Un avantage des compositions de l'invention est que, comme cela sera montré ci-après, par le choix des masses moléculaires de la matrice polymère, de la concentration de l'antibiotique, et de la dimension des particules dans le cas des poudres, ou encore par l'association de poudres ayant des caractéristiques différentes, il est possible d'adapter et de faire varier à volonté les profils de libération de l'antibiotique.

La demande de brevet EP-A-0.374.531 décrit une composition sous forme de microparticules contenant un agent thérapeutique dispersé dans une matrice comprenant un polymère biocompatible et biodégradable, cette composition étant destinée à traiter les maladies dentaires par insertion dans la poche parodontale. La poudre absorbe de l'eau et devient collante, ce qui favorise son maintien dans la poche parodontale. Cette poudre est constituée de microparticules obtenues de préférence par un procédé de séparation de phases. Il ne s'agit pas d'une poudre broyée.

La demande de brevet GB-A-2.091.554 concerne une composition implantable sous forme de tige pouvant pénétrer dans le corps du malade traité, cette composition comprenant une matrice d'un polymère tel qu'un poly(acide glycolique) ou un poly(acide lactique). Le polymère doit avoir un degré de cristallinité d'au moins 30 % dans le produit final. Il ne s'agit donc pas d'un polymère amorphe.

La demande de brevet EP-A-0.251.680 décrit une composition pour la libération contrôlée d'un composé pharmaceutique dans des fluides corporels tels que les larmes. Cette composition est constituée d'une matrice externe rapidement soluble dans lesdits fluides, et de microparticules biodégradables dispersées dans la matrice externes, ces microparticules contenant la substance active et étant capables de la libérer progressivement. Les microparticules, qui peuvent être de divers types, comprennent une matrice qui peut être choisie en un matériau tel que les poly(acides glycoliques), les poly(acides lactiques), les polyanhydrides, les poly(ortho-esters), etc. Il n'est pas prévu d'utiliser des poly(acides lactiques) amorphes. Il n'est pas prévu non plus d'utiliser une poudre broyée. La composition de cette demande de brevet européen est destinée à des applications dans la cavité oculaire.

La présente invention a donc pour objet une composition pharmaceutique implantable et biorésorbable à base de poly(acide lactique), destinée à mettre en oeuvre une antibiothérapie interne locale, caractérisée par le fait qu'elle comprend au moins un antibiotique soluble dans l'eau sous forme de particules de dimensions contrôlées, dispersées de façon homogène dans une matrice de poly(acide lactique) amorphe, et que ladite composition se présente sous la forme d'une poudre broyée ou d'un film. Un tel film est parfois désigné par l'expression "film mince" dans la description qui suit.

Dans la composition de l'invention, les particules d'antibiotique sont réparties de façon homogène (statistiquement), ce qui les différencie des microsphères par exemple, comme déjà signalé plus haut.

Ces particules d'antibiotique ont des dimensions inférieures à 100 µm, et en particulier comprises entre 0,01 et 50 µm.

Le déposant a découvert qu'une condition importante pour obtenir une dispersion homogène et une libération appropriée de la substance active dans une matrice de poly(acide lactique) est d'employer un poly(acide lactique) amorphe, car les matrices polymères semi-cristallines conduisent à des libérations trop rapides de la substance active et à des durées trop longues de dégradation du polymère.

Pour cela, il convient d'utiliser un polymère constitué d'un mélange de motifs dérivés des acides D- et L-lactiques, les proportions de chacun des motifs D- et L- étant suffisantes pour que la matrice soit amorphe. On peut utiliser en particulier les poly(acides lactiques) amorphes qui sont obtenus lorsque de 20 à 80 % des motifs qu'ils contiennent sont des motifs D-lactiques (les autres motifs étant bien entendu des motifs L-lactiques). On peut employer notamment les polymères obtenus au départ de mélanges racémiques d'acide D,L-lactique, ou de D,L-lactide, afin d'obtenir des polymères amorphes contenant des proportions égales des deux types de motifs.

L'invention a également pour objet un procédé de préparation d'une composition telle que définie précédemment. Ce procédé est caractérisé par le fait que l'on mélange lesdites particules d'antibiotique et ledit poly(acide lactique) de façon à obtenir une dispersion homogène, puis que, selon les méthodes connues, l'on met la dispersion obtenue sous la forme d'une poudre broyée ou d'un film mince.

Selon un premier mode de réalisation, la composition de l'invention se présente sous la forme d'une poudre obtenue par broyage d'une dispersion homogène de l'antibiotique dans une matrice de poly(acide lactique) amorphe ayant une masse moléculaire au moins égale à 10000, en particulier au moins égale à 30000. En effet, les polymères ayant une masse moléculaire trop faible ne conviennent pas pour être utilisés seuls : ils ont tendance à donner, avec l'antibiotique, soit des produits pâteux soit des poudres dont les profils de libération de l'antibiotique sont moins adaptés car ils conduisent notamment à une libération trop rapide de la substance active.

La masse moléculaire des poly(acides lactiques) amorphes utilisés selon l'invention dans la fabrication des poudres broyées est comprise généralement entre 10 000 et 200 000, et de préférence entre 20 000 et 300 000.

Il s'agit bien entendu d'une masse moléculaire moyenne, mesurée par exemple par chromatographie de perméation de gel, dans le dioxane, par rapport à des étalons de polystyrène.

On sait que les poly(acides lactiques) de masses moléculaires relativement élevées peuvent être obtenus notamment par polymérisation de lactide, par exemple par polymérisation du D,L-lactide. Il existe également des poly(acides lactiques) de masse moléculaire élevée utilisables qui sont des produits commerciaux.

Pour préparer la dispersion de l'antibiotique dans le polymère, on peut par exemple mélanger les particules d'antibiotique et le polymère, dans un solvant du polymère dans lequel l'antibiotique est insoluble. Après obtention d'une dispersion de l'antibiotique dans la solution du polymère, on évapore le solvant, par exemple dans un évaporateur rotatif pour favoriser l'homogénéité de la dispersion. On soumet ensuite la masse obtenue à un broyage, de façon à obtenir une poudre ayant par exemple des dimensions de particules comprises entre 0,1 et 1 mm.

Grâce à ce procédé, dans lequel on évite une dissolution suivie de reprécipitation de l'antibiotique, on peut contrôler les dimensions des particules d'antibiotique dans les poudres obtenues.

On peut ensuite soumettre le broyat obtenu, Si désiré, à un tamisage pour obtenir des poudres ayant la granulométrie souhaitée.

Des études de relargage de la substance antibiotique, en tampon phosphate isotonique de pH 7,4 à 37°C, ont montré que la vitesse de libération de l'antibiotique augmente lorsque les dimensions des particules de la composition diminuent. On peut éventuellement mettre à profit ce phénomène en utilisant un mélange d'au moins deux ensembles de particules ayant des dimensions moyennes différentes. Les petites particules permettront la libération rapide d'une dosage d'attaque de l'antibiotique, tandis que les particules plus grosses donneront une libération prolongée d'antibiotique. Par exemple, on utilisera un mélange de particules ayant des dimensions de 0,1 à 0,2 mm avec des particules ayant des dimensions moyennes de 0,5 à 1 mm, en proportions convenables.

Les études de relargage ont également montré que la vitesse de libération de l'antibiotique augmente avec la proportion d'antibiotique contenue dans la poudre. On peut ainsi, pour une poudre ayant des dimensions moyennes données, choisir la concentration de l'antibiotique de façon à obtenir une vitesse de libération appropriée. Par exemple, on choisira une poudre ayant une proportion d'antibiotique suffisante pour que la quantité d'antibiotique libérée, en tampon phosphate isotonique de pH 7,4 à 37°C, au bout de 24 heures, soit au moins égale à 20 % de la quantité initiale d'antibiotiques et soit inférieure à 70 % (en particulier inférieure à 50 %) de ladite quantité initiale.

Généralement, dans les poudres de l'invention, la concentration de l'antibiotique est comprise entre 5 et 30 % en poids, et choisie notamment en fonction des dimensions de particules de la poudre.

De même, les études de relargage de l'antibiotique en tampon phosphate ont montré que la vitesse de libération de l'antibiotique diminue lorsque la masse moléculaire du poly(acide lactique) augmente.

Ainsi, en utilisant le modèle du relargage de l'antibiotique en tampon phosphate, comme indiqué ci-dessus, on peut déterminer aisément, par de simples expériences de routine, les dimensions de particules des poudres, les concentrations d'antibiotique dans lesdites poudres, et les masses moléculaires du poly(acide lactique), qui donneront les profils souhaités de libération de l'antibiotique.

Comme déjà signalé ci-dessus, les matrices de poly(acide lactique) ayant des masses moléculaires faibles, notamment inférieures à 10000, conduisent à l'obtention de pâtes ou de poudres libérant trop rapidement l'antibiotique pour pouvoir être utilisées seules. Plus précisément, dans le cas d'un antibiotique aminé, l'antibiotique sous forme salifiée donne avec le poly(acide lactique) de faible masse moléculaire une pâte, tandis que l'antibiotique sous forme non salifiée donne une poudre. Par exemple, le poly(acide lactique) de faible masse moléculaire décrit à l'exemple 2 de la partie expérimentale ci-après, associé à 10 % de gentamycine base, donne une poudre qui, en tampon phosphate, libère en 24 heures environ 50 % de l'antibiotique qu'elle contient, puis le taux de libération d'antibiotique est faible dans les jours qui suivent. Une telle poudre ne pourra être utilisée seule, mais pourra être utilisée en association avec une poudre dont la matrice de poly(acide lactique) a une masse moléculaire et/ou une granulométrie suffisante pour que la libération de l'antibiotique qu'elle contient soit plus progressive.

Selon un second mode de réalisation, la composition de l'invention se présente sous la forme d'un film mince. Il est possible d'obtenir des films minces de poly(acide lactique), utilisables selon l'invention, par les procédés classiques de fabrication des films, notamment en mélangeant un poly(acide lactique) amorphe, de masse moléculaire moyenne élevée, par exemple supérieure à 20000, avec un poly(acide lactique) amorphe de masse moléculaire moyenne faible, en particulier inférieure à 5 000, ce dernier jouant le rôle de plastifiant. La proportion de poly(acide lactique) de faible masse moléculaire pourra être en particulier une proportion suffisante pour que la température de transition vitreuse du mélange de polymères soit inférieure à une température prédéterminée, par exemple à 37°C. En effet, la température de transition vitreuse diminue lorsque la proportion de polymère de faible masse moléculaire augmente.

Les films minces implantables de l'invention sont bien entendu des films souples (ou capables de se transformer rapidement en films souples au contact des fluides corporels présents au site d'implantation), de façon à éviter le traumatisme des tissus et à s'adapter à la forme du site chirurgical.

On peut régler facilement la souplesse du film, qui augmente avec la teneur en poly(acide lactique) de faible masse moléculaire.

On rappelle qu'il existe dans le commerce des poly(acides lactiques) ayant de faibles masses moléculaires. Les poly(acides lactiques) amorphes de faible masse moléculaire peuvent aussi être obtenus selon des méthodes connues, notamment par polycondensation de mélanges d'acides L- et D-lactiques, par exemple d'acide D,L-lactique.

Les études de relargage de l'antibiotique (en tampon phosphate, comme indiqué ci-dessus) à partir des films ont montré que, comme pour les poudres, la vitesse de libération de l'antibiotique augmente lorsque la proportion de l'antibiotique augmente.

Ces études ont également montré que la vitesse de libération de l'antibiotique augmente quand la proportion de poly(acide lactique) de faible masse moléculaire augmente.

Il est donc possible de régler, par de simples expériences de routine, les proportions convenables de poly(acide lactique) de faible masse moléculaire et/ou d'antibiotique pour obtenir la vitesse de libération d'antibiotique souhaitée.

Généralement, la proportion de poly(acide lactique) de faible masse moléculaire dans le mélange de polymères sera comprise entre 1 et 50 % en poids, et en particulier entre 5 et 40 %.

Pour un mélange de polymères donné, on choisira par exemple une proportion d'antibiotique suffisamment élevée pour que la quantité d'antibiotique libérée, en tampon phosphate isotonique de pH 7,4 à 37°C, au bout de 24 heures, soit au moins égale à 20 % de la quantité initiale, pour un film ayant une épaisseur initiale de 0,3 mm, ladite proportion d'antibiotique étant choisie suffisamment faible pour que ladite quantité libérée soit inférieure à 70 % de la quantité initiale.

On peut également, ou simultanément, jouer sur les proportions des polymères et choisir une proportion de polymère de faible masse moléculaire à la fois suffisamment élevée et suffisamment faible pour que la quantité d'antibiotique libérée, dans les conditions qui viennent d'être indiquées, soit située dans la gamme déjà mentionnée.

Pour préparer une composition sous forme de film, on peut opérer comme dans le cas des poudres, c'est-à-dire mélanger les particules d'antibiotique et le poly(acide lactique), dans un solvant du polymère dans lequel l'antibiotique est insoluble, puis évaporer le solvant, de préférence lentement de façon à éviter la formation de bulles en surface. Le poly(acide lactique) est ici le mélange de polymères de haute masse moléculaire et de faible masse moléculaire. L'épaisseur du film peut être ensuite ajustée par calandrage. On peut également mélanger les particules d'antibiotique par malaxage avec le polymère, puis former un film par calandrage.

On prépare ainsi des films ayant par exemple une épaisseur pouvant varier dans la gamme de 0,05 - 1 mm.

De façon à favoriser les échanges chimiques, la cicatrisation des tissus et la circulation sanguine (revascularisation) au site d'implantation, on peut transformer le film en un film perforé, muni d'une pluralité de perforations. La surface des perforations représente par exemple de 10 à 70 % de la surface totale du film, selon l'épaisseur du film. La surface des perforations pourra être d'autant plus importante que le film est plus épais.

L'antibiotique présent dans les compositions de l'invention est de préférence un antibiotique à large spectre. Il est toutefois possible d'associer plusieurs antibiotiques, non seulement antibactériens, mais aussi antifongiques. Bien entendu, on choisira de préférence des antibiotiques non ou rarement allergisants. On peut citer par exemple les aminoglycosides comme la gentamycine, la tobramycine, l'amikacine, la nétilmicine, la néomycine ; les macrolides comme l'érythromycine ; les antibiotiques polypeptidiques comme la polymyxine B, la bacitracine, la gramicidine ; la lincomycine et ses dérivés, notamment la clindamycine ; les rifamycines, en particulier la rifampicine ; les tétracyclines ; les 5-nitro imidazoles tels que le métronidazole et le tinidazole ; le ketoconazole, la nystatine, la griséofulvine, l'amphothéricine ; ou encore l'acide fusidique (sous forme de sel soluble), la spectinomycine ou la vancomycine.

Les antibiotiques comportent souvent des groupements amine salifiables. L'antibiotique présent dans la composition de l'invention peut être sous forme libre ou salifiée. Les sels sont bien entendu des sels compatibles avec une utilisation pharmaceutique. On citera par exemple les sulfates, les chlorhydrates, etc..., ou encore des sels obtenus avec des polyanions, de préférable biorésorbables, tels que le poly(acide malique) qui est décrit notamment dans les brevets US 4265247 et 4320753. De même, les antibiotiques contenant des groupements carboxyliques peuvent être utilisés sous forme de sels, par exemple de sels alcalins (sodium, potassium).

Il convient de noter que lorsqu'un antibiotique aminé n'est pas sous forme salifiée, il peut s'associer avec les groupements carboxyliques du poly(acide lactique) (groupements carboxyliques terminaux ou groupements carboxyliques apparaissant à la suite de la dégradation du polyacide). Généralement, une telle association aura tendance à diminuer la vitesse de libération de l'antibiotique et/ou à limiter cette libération.

L'invention a également pour objet un procédé de traitement thérapeutique destiné à mettre en oeuvre une antibiothérapie interne locale post-chirurgicale, caractérisée par le fait que l'on implante dans la cavité opératoire une quantité efficace d'une composition sous forme de poudre broyée ou de film mince, telle que définie précédemment.

La composition de l'invention est administrée comme indiqué ci-dessus, notamment chez l'homme, par mise en place à la fin de l'opération chirurgicale, avant suture de la plaie. Les doses utilisées sont celles qui permettent d'obtenir la libération à doses efficaces de l'antibiotique, ces doses efficaces étant connues. On peut donc déterminer dans chaque cas, (par exemple à l'aide du modèle d'étude en tampon phosphate mentionné ci-dessus) les doses de composition implantable qu'il convient d'utiliser.

Bien entendu, on peut associer l'utilisation d'une composition sous forme de poudre et d'une composition sous forme de film, la composition sous forme de poudre étant choisie par exemple pour permettre une libération plus rapide de l'antibiotique dans les premières heures suivant l'implantation.

Les exemples suivants illustrent l'invention.

### EXEMPLE 1 : Préparation de poudres broyées

On prépare un polymère d'acide lactique par polymérisation de D,L-lactide. Pour cela, on effectue une polymérisation en masse, en présence de 0,05 % en poids de zinc, en ballon scellé à 140°C, sous agitation pendant 3 semaines. Après refroidissement, on dissout le produit réactionnel dans le chloroforme ou l'acétone, et on ajoute un alcool (méthanol ou éthanol) pour précipiter le polymère formé, tandis que les monomères résiduels et les poly(acides lactiques) de bas poids moléculaires formés restent en solution. Le polymère est ensuite séché pendant une semaine sous vide à 40°C.

Le polymère obtenu a une masse moléculaire moyenne en nombre de 160 000 et une masse moléculaire moyenne en poids de 280 000.

Le spectre de diffraction X montre l'absence de domaine cristallin.

Ce polymère amorphe a une température de transition vitreuse, déterminée par analyse thermique différentielle, de 44°C environ.

On dissout 5 g de ce polymère dans 100 ml d'acétone. On ajoute une quantité suffisante de sulfate de gentamycine pour que le mélange final ait une teneur de 10 % en poids en sulfate de gentamycine. Le mélange est alors évaporé à sec à l'aide d'un évaporateur rotatif, puis est séché sous pression réduite à 40°C.

Le produit séché est ensuite broyé cryogéniquement (à la température de l'azote liquide). Après broyage, il se présente sous la forme de particules solides. On sépare par tamisage trois fractions ayant des tailles de particules comprises respectivement entre 0,125-0,25 mm (petites tailles), 0,25-0,5 mm (tailles moyennes) et 0,5-1 mm (grandes tailles).

Les études de relargage de l'antibiotique en tampon phosphate, dans les conditions indiquées précédemment, ont montré que les poudres de petite taille libèrent dans les 24 heures plus de 50 % de l'antibiotique qu'elles contiennent. La libération est ensuite très faible. Les poudres de taille moyenne libèrent environ 40 % de l'antibiotique en 24 heures, et 55 % environ dans les dix premiers jours. Les poudres de grande taille libèrent 25 à 30 % de l'antibiotique le premier jour, pour arriver à une libération de 50 % au bout de 10 jours.

De façon analogue, on a préparé des poudres contenant respectivement 20 % et 30 % de sulfate de gentamycine, ainsi que des poudres contenant l'un des antibiotiques suivants : tobramycine, néomycine, bacitracine, clindamycine et rifampicine.

On a également préparé de façon analogue une poudre contenant 20 % en poids d'un complexe de poly(acide malique) et de gentamycine, ce complexe ayant été préparé au départ du sel de sodium d'un poly(acide malique) (MM : 20000) dissous dans de l'eau, auquel on ajoute un excès de sulfate de gentamycine.

### EXEMPLE 2 : Préparation de films

De façon analogue à celle décrite à l'exemple 1, on a préparé un poly(acidelactique) ayant les caractéristiques suivantes :
- Mn = 170 000
- Mp = 280 000
- Température de transition vitreuse : 35°C
- Absence de domaine cristallin.

Par ailleurs, on a préparé un acide polylactique de faible poids moléculaire par polycondensation de l'acide D,L-lactique à 140°C sous vide pendant 3 jours. Après refroidissement, le produit réactionnel est dissous dans l'acétone, et on précipite le polymère par addition d'eau à la solution, tandis que les monomères résiduels restent en solution. Le polymère est ensuite séché pendant une semaine sous vide.

Il présente les caractéristiques suivantes :
- Mn = 2 000
- Mp = 2 600

Ce polymère se présente sous la forme d'une pâte visqueuse et collante. Sa température de transition vitreuse est de l'ordre de 0°C.

Comme à l'exemple 1, les polymères, en proportions convenables, sont dissous dans l'acétone et on ajoute une quantité calculée de sulfate de gentamycine. Le mélange est alors coulé dans une boîte de Petri revêtue de Téflon.

Le solvant est évaporé à température ambiante, sous la pression atmosphérique, pour éviter la formation de bulles en surface. Le séchage est ensuite terminé sous pression réduite. Finalement, on effectue le calandrage avec une calandre à rouleau chromé.

On a ainsi préparé des films ayant une épaisseur de 0,3 mm environ dont la proportion de poly(acide lactique) de faible masse moléculaire est de 10, 20 ou 30 %, par rapport à la masse totale de polymères, ces films contenant en poids, 10 % ou 20 % de sulfate de gentamycine.

On a préparé de façon analogue des films dans lesquels le polymère de faible masse moléculaire est un polymère obtenu par polycondensation d'un mélange d'acides D-lactique et L-lactique contenant 70 % d'acide D-lactique.

Les études de relargage en tampon phosphate montrent que le phénomène de "burst" est atténué dans le cas des films. Au bout de 24 heures, environ 20 % de l'antibiotique sont libérés dans le cas d'une matrice à 10 % ou 20 % de polymère de faible masse moléculaire, pour les films contenant 10 % de sulfate de gentamycine. Ensuite, la libération de l'antibiotique se poursuit régulièrement, d'autant plus rapidement que la teneur en polymère de faible masse moléculaire est plus élevée. Dans le cas de la matrice contenant 20 % de polymère de faible masse moléculaire, la libération de l'antibiotique atteint 50 % entre 20 et 30 jours environ.

De façon analogue, on a préparé des films contenant l'un des antibiotiques suivants : oxytétracycline, doxycycline, érythromycine, métronidazole, ketoconazole, clindamycine et chloramphénicol.

### EXEMPLE 3 : Etude in vivo

### Implantation de particules

Des particules ayant des tailles comprises entre 0,5 et 1 mm, contenant 10 % de sulfate de gentamycine, obtenues à l'exemple 1 ont été implantées dans la partie postérieure du muscle paravertébral de lapins à raison de deux sites d'implantations par animal, de chaque côté de la colonne vertébral. Les quantités implantées étaient de 100 mg.

On dose régulièrement la quantité de gentamycine excrétée dans les urines, selon une méthode analogue à celle décrite par Gambardella et al., Journal of Chromatography, 348, 229-240 (1985).

La quantité excrétée est importante les deux premiers jours (respectivement 2 et 3 mg/jour aux premier et deuxième jours) puis décroît lentement jusqu'au quinzième jour après l'implantation.

Au bout de 14 jours, on note généralement aux sites d'implantation une petite zone de nécrose des tissus musculaires.

Par comparaison, le sulfate de gentamycine implanté seul (10 mg) donne des zones de nécrose étendues ; l'excrétion urinaire est très élevée le premier jour (7 mg/jour) pour descendre à 1 mg/jour le deuxième jour et devenir nulle le huitième jour.

### Implantation de films

Les films ont été implantés, comme précédemment, chez des lapins, à raison de 75 mg par site d'implantation.

Les films utilisés, obtenus comme décrit à l'exemple 2, sont des mélanges de polymères contenant respectivement 10 %, 20 % ou 30 % du poly(acide D,L-lactique) de faible masse moléculaire, tous les films étudiés contenant 10 % de sulfate de gentamycine.

Cette étude a duré 10 jours.

Comme dans le cas des poudres, l'excrétion urinaire de gentamycine est élevée les deux premiers jours puis décroît les jours suivants. Elle est toujours présente après 10 jours, alors qu'avec le sulfate de gentamycine implanté seul, elle devient pratiquement nulle au septième jour. Par ailleurs, les quantités d'antibiotique excrétées sont d'autant plus importantes que la proportion de polymère de faible masse moléculaire est importante.

Au dixième jour, les nécroses sont nettement plus importantes chez les lapins ayant reçu le sulfate de gentamycine seul. En outre, on remarque que l'épaisseur des zones nécrosées autour des implants est environ deux fois plus faible que celle des zones nécrosées du canal d'implantation, ce qui suggère que la nécrose est essentiellement due au traumatisme chirurgical lors de l'insertion des films.

En conclusion, les études in vivo ont montré, avec les compositions implantables de l'invention, la libération progressive de l'antibiotique, après un burst plus ou moins important, notamment selon la nature de l'implant.

D'une façon générale, des études in vivo ont confirmé la validité des résultats obtenus avec le modèle de libération in vitro en tampon phosphate.

La toxicité est moins importante qu'avec le sulfate de gentamycine implanté seul, et les réactions tissulaires sont relativement faibles, ce qui confirme l'excellente biocompatibilité des compositions implantables de l'invention.

## Revendications

1. Composition pharmaceutique implantable et biorésorbable à base de poly(acide lactique), destinée à mettre en oeuvre une antibiothérapie interne locale, caractérisée par le fait qu'elle comprend au moins un antibiotique soluble dans l'eau sous forme de particules de dimensions contrôlées, inférieures à 100 µm, dispersées de façon homogène dans une matrice de poly(acide lactique) amorphe, et que ladite composition se présente sous la forme d'une poudre broyée ou d'un film.

2. Composition selon la revendication 1, caractérisée par le fait que ledit poly(acide lactique) est constitué d'un mélange de motifs dérivés des acides D- et L-lactiques, les proportions de chacun des motifs D- et L- étant suffisantes pour que ledit poly(acide lactique) soit amorphe.

3. Composition selon la revendication 2, caractérisée par le fait que ledit poly(acide lactique) contient de 20 à 80 % de motifs D-lactiques.

4. Composition selon la revendication précédente, caractérisée par le fait que ledit poly(acide lactique) contient en proportions égales des motifs dérivés des acides D- et L-lactiques.

5. Composition selon l'une quelconque des revendications précédentes, sous forme de poudre broyée, caractérisée par le fait que ledit poly(acide lactique) a une masse moléculaire au moins égale à 10000.

6. Composition selon la revendication précédente, caractérisée par le fait que ladite masse moléculaire est comprise entre 10 000 et 300 000.

7. Composition selon l'une quelconque des revendications 4 à 6, caractérisée par le fait que ladite poudre a des dimensions de particules comprises entre 0,1 et 1 mm.

8. Composition selon la revendication précédente, caractérisée par le fait que ladite poudre est constituée d'un mélange de particules ayant des dimensions de 0,1 à 0,2 mm avec des particules ayant des dimensions de 0,5 à 1 mm.

9. Composition selon l'une quelconque des revendications 4 à 8, caractérisée par le fait que la proportion d'antibiotique qu'elle contient est suffisante pour que la quantité d'antibiotique libérée, en tampon phosphate isotonique de pH 7,4 à 37°C, au bout de 24 heures, soit au moins égale à 20 % de la quantité initiale d'antibiotiques et soit inférieure à 70 %, et en particulier inférieure à 50 %, de ladite quantité initiale.

10. Composition selon la revendication précédente, caractérisée par le fait que ladite proportion est comprise entre 5 et 30 % en poids.

11. Composition selon l'une quelconque des revendications 1 à 3, sous la forme d'un film, caractérisée par le fait que ladite matrice est constituée d'un mélange d'un poly(acide lactique) amorphe de masse moléculaire supérieure à 20 000, avec un poly(acide lactique) amorphe de faible masse moléculaire, inférieure à 5 000.

12. Composition selon la revendication précédente, caractérisée par le fait que la proportion de poly(acide lactique) de faible masse moléculaire est suffisante pour que la température de transition vitreuse du mélange de polymères soit inférieure à 37°C.

13. Composition selon la revendication 11 ou 12, caractérisée par le fait que la proportion d'antibiotique et/ou la proportion de poly (acide lactique) de faible masse moléculaire sont suffisamment élevées pour que la quantité d'antibiotique libérée, en tampon phosphate isotonique de pH 7,4, à 37°C, au bout de 24 heures, soit au moins égale à 20 % de la quantité initiale, pour un film ayant une épaisseur initiale de 0,3 mm, et que lesdites proportions sont suffisamment faibles pour que ladite quantité libérée soit inférieure à 70 % de la quantité initiale.

14. Composition selon l'une quelconque des revendications 11 à 13, caractérisée par le fait que ledit film a une épaisseur dans la gamme de 0,05-1 mm.

15. Composition selon l'une quelconque des revendications 11 à 14, caractérisée par le fait que ledit film est muni d'une pluralité de perforations.

16. Composition selon la revendication précédente, caractérisée par le fait que la surface des perforations représente de 10 à 70 % de la surface totale du film.

17. Composition selon l'une quelconque des revendications précédentes, caractérisée par le fait que lesdites particules d'antibiotique ont des dimensions comprises entre 0,01 et 50 µm.

18. Composition selon l'une quelconque des revendications précédentes, caractérisée par le fait que ledit antibiotique est présent sous forme de sel.

19. Procédé de préparation d'une composition telle que définie dans l'une quelconque des revendications précédentes, caractérisé par le fait que l'on mélange lesdites particules d'antibiotique et ledit poly(acide lactique) de façon à obtenir une dispersion homogène, puis que, selon les méthodes connues, l'on met la dispersion obtenue sous la forme d'une poudre broyée ou d'un film étant entendu que pour préparer la composition sous forme de poudre broyée, le poly(acide lactique) de départ a une masse moléculaire au moins égale à 10000, et étant entendu que pour préparer la composition sous forme de film, le poly(acide lactique) de départ est au mélange d'un poly(acide lactique) amorphe de masse moléculaire supérieure à 20000 avec un poly(acide lactique) amorphe de masse moléculaire inférieure à 5000, ce dernier jouant le rôle de plastifiant.

20. Procédé selon la revendication précédente, caractérisé par le fait que l'on mélange les particules d'antibiotique et le poly(acide lactique), dans un solvant du polymère dans lequel l'antibiotique est insoluble, puis on évapore le solvant et met la composition obtenue sous la forme désirée, par broyage ou calandrage.

21. Procédé selon la revendication 19, caractérisé par le fait que pour préparer un film, on mélange les particules d'antibiotique, par malaxage, avec le poly(acide lactique), puis on met la composition obtenue sous forme de film par calandrage.

22. Utilisation, dans la préparation d'une composition implantable biorésorbable destinée à mettre en oeuvre une antibiothérapie interne locale, d'une dispersion de particules d'au moins un antibiotique soluble dans l'eau, dans une matrice d'acide polylactique amorphe, lesdites particules ayant des dimensions contrôlées, inférieures à 100 µm, ladite composition se présentant sous la forme d'une poudre broyée ou d'un film.

23. Utilisation selon la revendication précédente, caractérisée par le fait que ladite composition est telle que définie dans l'une quelconque des revendications 2 à 18.

## Claims

1. Implantable, bioresorbable pharmaceutical composition based on poly(lactic acid), intended to carry out a local internal antibiotic therapy, characterized in that it comprises at least one water-soluble antibiotic in the form of particles of controlled size, smaller than 100 µm, homogeneously dispersed in an amorphous poly(lactic acid) matrix, and in that the said composition is in the form of a ground powder or a film.

2. Composition according to Claim 1, characterized in that the said poly(lactic acid) consists of a mixture of units derived from D- and L-lactic acids, the proportions of each of the D and L units being sufficient for the said poly(lactic acid) to be amorphous.

3. Composition according to Claim 2, characterized in that the said poly(lactic acid) contains from 20 to 80% D-lactic units.

4. Composition according to the preceding claim, characterized in that the said poly(lactic acid) contains equal proportions of units derived from D- and L-lactic acids.

5. Composition according to any one of the preceding claims, which is in the form of a ground powder, characterized in that the said poly(lactic acid) has a molecular mass at least equal to 10,000.

6. Composition according to the preceding claim, characterized in that the said molecular mass is between 10,000 and 300,000.

7. Composition according to any one of Claims 4 to 6, characterized in that the said powder has particles between 0.1 and 1 mm in size.

8. Composition according to the preceding claim, characterized in that the said powder consists of a mixture of particles from 0.1 to 0.2 mm in size with particles from 0.5 to 1 mm in size.

9. Composition according to any one of Claims 4 to 8, characterized in that the proportion of antibiotic which it contains is sufficient for the amount of antibiotic released, in isotonic phosphate buffer of pH 7.4 at 37°C, at the end of 24 hours, to be at least equal to 20% of the initial amount of antibiotics and to be less than 70%, and in particular less than 50%, of the said initial amount.

10. Composition according to the preceding claim, characterized in that the said proportion is between 5 and 30% by weight.

11. Composition according to any one of Claims 1 to 3, in the form of a film, characterized in that the said matrix consists of a mixture of an amorphous poly(lactic acid) of molecular mass greater than 20,000, with an amorphous poly(lactic acid) of low molecular mass, less than 5000.

12. Composition according to the preceding claim, characterized in that the proportion of poly(lactic acid) of low molecular mass is sufficient for the glass transition temperature of the polymer mixture to be less than 37°C.

13. Composition according to Claim 11 or 12, characterized in that the proportion of antibiotic and/or the proportion of poly(lactic acid) of low molecular mass are sufficiently high for the amount of antibiotic released, in isotonic phosphate buffer of pH 7.4, at 37°C, at the end of 24 hours, to be at least equal to 20% of the initial amount, for a film having an initial thickness of 0.3 mm, and in that the said proportions are sufficiently low for the said amount released to be less than 70% of the initial amount.

14. Composition according to any one of Claims 11 to 13, characterized in that the said film has a thickness in the range 0.05-1 mm.

15. Composition according to any one of Claims 11 to 14, characterized in that the said film is provided with a plurality of perforations.

16. Composition according to the preceding claim, characterized in that the area of the perforations represents from 10 to 70% of the total area of the film.

17. Composition according to any one of the preceding claims, characterized in that the said antibiotic particles are between 0.01 and 50 µm in size.

18. Composition according to any one of the preceding claims, characterized in that the said antibiotic is present in the form of a salt.

19. Process for the preparation of a composition as defined in any one of the preceding claims, characterized in that the said antibiotic particles and the said poly(lactic acid) are mixed together so as to obtain a homogeneous dispersion and the dispersion obtained is then formed into a ground powder or a film, according to the known methods, it being understood that in order to prepare the composition in the form of ground powder, the starting poly(lactic acid) has a molecular mass at least equal to 10,000, and it being understood that in order to prepare the composition in the form of a film, the starting poly(lactic acid) is a mixture of an amorphous poly(lactic acid) of molecular mass greater than 20,000 with an amorphous poly(lactic acid) of molecular mass less than 5000, the latter acting as a plasticizer.

20. Process according to the preceding claim, characterized in that the antibiotic particles and the poly(lactic acid) are mixed together, in a solvent for the polymer in which the antibiotic is insoluble, the solvent is then evaporated off and the composition obtained is made into the desired form, by grinding or calendering.

21. Process according to Claim 19, characterized in that in order to prepare a film, the antibiotic particles are mixed together, by blending, with the poly(lactic acid) and the composition obtained is then made into a film by calendering.

22. Use, in the preparation of an implantable, bioresorbable composition intended to carry out a local internal antibiotic therapy, of a particle dispersion of at least one water-soluble antibiotic, in an amorphous polylactic acid matrix, the said particles having controlled sizes, smaller than 100 µm, the said composition being in the form of a ground powder or a film.

23. Use according to the preceding claim, characterized in that the said composition is as defined in any one of Claims 2 to 18.

## Patentansprüche

1. Implantierbare und bioresorbierbare pharmazeutische Zusammensetzung auf der Basis von Poly-(milchsäure), bestimmt zur Durchführung einer internen lokalen antibiotischen Therapie, dadurch gekennzeichnet, daß sie mindestens ein in Wasser lösliches Antibiotikum in Form von Teilchen von kontrollierten Abmessungen unter 100 µm enthält, die in homogener Weise in einer Matrix aus amorpher Poly-(milchsäure) dispergiert sind, und daß die Zusammensetzung in Form eines gemahlenen Pulvers oder eines Films vorliegt.

2. Zusammensetzung nach Anspruch 1, dadurch gekennzeichnet, daß die Poly-(milchsäure) aus einer Mischung von von D- und L-Milchsaure abgeleiteten Grundeinheiten besteht, wobei die Anteile der jeweiligen D- und L-Grundeinheiten ausreichen, daß die Poly-(milchsäure) amorph ist.

3. Zusammensetzung nach Anspruch 2, dadurch gekennzeichnet, daß die Poly-(milchsäure) 20 bis 80% D-Milchsäure-Grundeinheiten enthält.

4. Zusammensetzung nach dem vorstehenden Anspruch, dadurch gekennzeichnet, daß die Poly-(milchsäure) gleiche Anteile an von D- und von L-Milchsäure abgeleiteten Grundeinheiten enthält.

5. Zusammensetzung nach einem der vorstehenden Ansprüche in Form eines gemahlenen Pulvers, dadurch gekennzeichnet, daß die Poly-(milchsäure) eine Molekülmasse von mindestens 10 000 aufweist.

6. Zusammensetzung nach dem vorstehenden Anspruch, dadurch gekennzeichnet, daß die Molekülmasse 10 000 bis 300 000 beträgt`

7. Zusammensetzung nach einem dar Ansprüche 4 bis 6, dadurch gekennzeichnet, daß das Pulver Teilchenabmessungen von 0,1 bis 1 mm aufweist.

8. Zusammensetzung nach dem vorstehenden Anspruch, dadurch gekennzeichnet, daß das Pulver aus einer Mischung von Teilchen, die Abmessungen von 0,1 bis 0,2 mm aufweisen, mit Teilchen, die Abmessungen von 0,5 bis 1 mm aufweisen, besteht.

9. Zusammensetzung nach einem der Ansprüche 4 bis 8, dadurch gekennzeichnet, daß der Anteil des enthaltenen Antibiotikums ausreicht, um in isotonischem Phosphatpuffer vom pH-Wert 7,4 bei 37°C innerhalb von 24 Stunden eine Antibiotikummenge freizusetzen, die mindestens 20% der ursprünglichen Menge der Antibiotika entspricht und weniger als 70% und insbesondere weniger als 50% dieser ursprünglichen Menge betragt.

10. Zusammensetzung nach dem vorstehenden Anspruch, dadurch gekennzeichnet, daß der Anteil 5 bis 30 Gew.-% beträgt.

11. Zusammensetzung nach einem der Ansprüche 1 bis 3 in Form eines Films, dadurch gekennzeichnet, daß die Matrix aus einer Mischung von amorpher Poly-(milchsäure), die eine Molekülmasse von mehr als 20 000 aufweist, mit einer amorphen Poly-(milchsäure), die eine geringe Molekülmasse von weniger als 5000 aufweist, besteht.

12. Zusammensetzung nach dem vorstehenden Anspruch, dadurch gekennzeichnet, daß der Anteil der Poly-(milchsäure) mit geringer Molekülmasse ausreicht, daß die Glasübergangstemperatur der Mischung der Polymeren unter 37°C liegt.

13. Zusammensetzung nach Anspruch 11 oder 12, dadurch gekennzeichnet, daß der Anteil des Antibiotikums und/oder der Anteil der Poly-(milchsäure) von geringer Molekülmasse ausreichen, um in isotonischem Phosphatpuffer vom pH-Wert 7,4 bei 37°C innerhalb von 24 Stunden eine Antibiotikummenge von mindestens 20% der ursprünglichen Menge bei einem Film mit einer anfänglichen Dicke von 0,3 mm freizusetzen, und daß die genannten Anteile ausreichend nieder sind, um weniger als 70% der Anfangsmenge freizusetzen.

14. Zusammensetzung nach einem der Ansprüche 11 bis 13, dadurch gekennzeichnet, daß der Film eine Dicke im Bereich von 0,05-1 mm aufweist.

15. Zusammensetzung nach einem der Ansprüche 11 bis 14, dadurch gekennzeichnet, daß der Film mit einer Mehrzahl von Perforationen versehen ist.

16. Zusammensetzung nach dem vorstehenden Anspruch, dadurch gekennzeichnet, daß die Oberfläche der Perforationen 10 bis 70% der Gesamtfläche des Films ausmacht.

17. Zusammensetzung nach einem der vorstehenden Ansprüche, dadurch gekennzeichnet, daß die Teilchen dem Antibiotikums Abmessungen im Bereich von 0,01 bis 50 µm aufweisen.

18. Zusammensetzung nach einem der vorstehenden Ansprüche, dadurch gekennzeichnet, daß das Antibiotikum in Form eines Salzes vorliegt.

19. Verfahren zur Herstellung einer Zusammensetzung gemäß der Definition in einem der vorstehenden Ansprüche, dadurch gekennzeichnet, daß man die genannten Teilchen des Antibiotikums und die Poly-(milchsäure) unter Bildung einer homogenen Dispersion vermischt und anschließend die erhaltene Dispersion nach bekannten Verfahren in die Form eines gemahlenen Pulvers oder eines Films bringt, wobei zur Herstellung der Zusammensetzung in Form eines gemahlenen Pulvers die als Ausgangsmaterial verwendete Poly-(milchsäure) eine Molekülmasse von weniger als 10 000 aufweist und wobei zur Herstellung der Zusammensetzung in Form eines Films die als Ausgangsmaterial verwendete Poly-(milchsäure) in Form einer Mischung einer amorphen Poly-(milchsäure), die eine Molekülmasse von mehr als 20 000 aufweist, mit einer amorphen Poly-(milchsäure), die eine Molekülmasse von Weniger als 5000 aufweist, vorliegt, wobei das letztgenannte Produkt die Funktion eines Weichmachers ausübt.

20. Verfahren nach dem vorstehenden Anspruch, dadurch gekennzeichnet, daß man die Teilchen des Antibiotikums und die Poly-(milchsäure) in einem Lösungsmittel für das Polymere, in dem das Antibiotikum unlöslich ist, vermischt, anschließend das Lösungsmittel abdampft und die erhaltene Zusammensetzung durch Mahlen oder Kalandrieren in die gewünschte Form bringt.

21. Verfahren nach Anspruch 19, dadurch gekennzeichnet, daß man zur Herstellung eines Films die Teilchen des Antibiotikums durch Verkneten mit der Poly-(milchsäure) vermischt und anschließend die erhaltene Zusammensetzung durch Kalandrieren in die Form eines Films bringt.

22. Verwendung einer Dispersion von Teilchen mit mindestens einem in Wasser löslichen Antibiotikum in einer Matrix aus amorpher Poly-(milchsäure) zur Herstellung einer implantierbaren, bioresorbierbaren Zusammensetzung für die Durchführung einer internen lokalen antibiotischen Therapie, wobei die Teilchen kontrollierte Abmessungen von weniger als 100 µm aufweisen und wobei die Zusammensetzung in Form eines gemahlenen Pulvers oder eines Films vorliegt.

23. Verwendung nach dem vorstehenden Anspruch, dadurch gekennzeichnet, daß die Zusammensetzung der Definition in einem der Ansprüche 2 bis 18 entspricht.
